# EUROPEAN PATENT APPLICATION

(11) **EP 2 942 069 A1**
(43) Date of publication of application: **11.11.2015**
(21) Application number: 15167008.0
(22) Date of filing: 08.05.2015
(51) Int. Cl.: A61L 27/36, A61L 2/00, A61F 2/28

(54) **BONE MATERIAL REUSE PROCESS**

(30) Priority: 09.05.2014 NL 2012797
(71) Applicant: Tournois Dynamic Innovations B.V., 6708 PW Wageningen (NL)
(72) Inventor: van Domselaar, Mark, 6708 PW Wageningen (NL); Hulleman, Stéphan, Henrick, Dick, 6708 PW Wageningen (NL)
(74) Representative: Ellens, Andries

(57) **Abstract**

The invention provides a process, especially for the efficient reuse of human bone material, the process comprising (i) screening a bone part on the suitability for reuse in a human body, (ii) packaging the bone part, (iii) subjecting the bone part to a high pressure process, wherein the bone part enclosed by a package is subjected to a pressure selected from the range of 300-1000 MPa for a period of time selected from the range of 1-60 minutes, wherein the package comprises a closed polymeric package containing the bone part and an aqueous liquid, and (iv) optionally further packaging the thus obtained high pressure process processed bone part, wherein the bone part has one or more dimensions selected from the range of 1-65 cm.

## Description

### FIELD OF THE INVENTION

The invention relates to a process for reuse of (human) bone material. The invention further relates to such bone material per se as well as to a packaged bone part obtainable with such process.

### BACKGROUND OF THE INVENTION

The use of implants and their pretreatment before implantation is known in the art. US6652818, for instance, describes a method for pooling of tissues for treatment prior to implantation into a recipient in need thereof. In one embodiment, the method includes perfusion of a porous implant which achieves efficient interpenetration of desired factors into and removal of undesirable factors from the pores of the implant, cleaning of the implant, efficient passivation of the implant (inactivation of pathogens, microorganisms, cells, viruses and the like and reduction in antigenicity thereof), and the novel implant produced by such treatment. The process presents a system wherein the rate of pressure cycling, the fact of pressure cycling, and the amplitude of pressure cycling, results in highly cleaned tissues and other implants for implantation.

### SUMMARY OF THE INVENTION

The current gold standard for sterilization by gamma irradiation and thermal or chemical inactivation of potentially infected autografts and allografts considered for tissue regeneration and reconstruction was recently clearly associated with deterioration of the mechanical, physical and biological properties of the bony implant. Sterilization of bone and soft tissue allograft at present is mainly performed using extracorporeal irradiation or autoclaving, followed by subzero preservation in a bone bank.

Another problem with prior art methods is that a microbial screening of bone parts is applied wherein superficial bacterial contamination of the bone parts cannot efficiently be identified. Some bone parts may pass the microbial screening, which may result in undesired infection of the recipient (human or animal) of the bone material or bone graft.

Hence, it is an aspect of the invention to provide an alternative process and/or an alternative bone reconstruction material, which preferably further at least partly obviate one or more of above-described drawbacks.

In a first aspect, the invention provides a process, especially for the efficient reuse of bone material, especially human bone material, the process comprising (i) screening a bone part (optionally external from a (donor) body), on the suitability for reuse in a human or animal body, especially a human body, (ii) optionally packaging the bone part (after removal from the (human or animal) body) (thereby providing a packaged bone part), (iii) subjecting the bone part to a high pressure process (HPP) (or high hydrostatic pressure process (HHP)), wherein the bone part, especially the bone part being enclosed by the package, is subjected to a pressure of at least 300 MPa, even more especially at least 450 MPa, such as in the range of 300-1000 MPa, even more especially selected from the range of 300-800 MPa, such as especially 450-800 MPa, especially for a period of time selected from the range of 1-60 minutes, such as at least 2 minutes, wherein the package especially comprises a closed polymeric package containing the bone part and an aqueous liquid, and (iv) optionally (further) packaging the thus obtained high pressure process processed (packaged) bone part. Hence, especially the bone part is packaged in the package, the package especially being impermeable to water, before the high pressure process, and is subject to this high pressure process while being packaged in the (water impermeable) package, such as a plastic bag.

In a further aspect, the invention provides a process, especially for the efficient reuse of bone material, especially human bone material, the process comprising (i) screening a bone part (optionally external from a (donor) body), on the suitability for reuse in a human or animal body, especially a human body, (iia) disintegrating the bone part into (particulate) bone material (after removal from the (human or animal) body), (iib) optionally packaging the bone material, (iii) subjecting the bone material to a high pressure process (HPP) (or high hydrostatic pressure process (HHP)), wherein the bone material, especially the bone material being enclosed by the package (which is especially impermeable to water), is subjected to a pressure of at least 300 MPa, even more especially at least 450 MPa, such as in the range of 300-1000 MPa, even more especially selected from the range of 450-800 MPa, especially for a period of time selected from the range of 1-60 minutes, such as at least 2 minutes, and (iv) optionally (further) packaging the thus obtained high pressure process processed bone material.

Further, these processes may especially comprise (v) transporting the packaged bone part (or bone material) to a (further) processing stage (such as an operating room). Further, assuming a bone part, these processes may further comprise (vi) (optionally removing the package) and (optionally) disintegrating at least part of the bone part into bone material, especially bone reconstruction material, which can be used as graft or as bone filler material. When using as graft, it may still be necessary to modify the bone part to fit to the recipient (acceptor body). Hence, in such instance disintegrating at least part of the bone part may be executed. In the case of an autograft, disintegration (even at least part) may not always be necessary, dependent upon the type of implant.

Especially, in an embodiment the process further involves removing the package and disintegrating at least part of the bone part into bone material (i.e. bone reconstruction material), especially having dimensions smaller than 1 cm for use as bone filler material, though other dimensions may also be possible. Hence, in another embodiment disintegrating may also include grinding. However, disintegrating may also refer to processing into bone (material) flakes (which may also be used as bone filler material).

With such process, the screening process may e.g. be easier, because one may only screen on severe bone deteriorating effects, such as bone cancer or Osteomyelitis. Hence, the screening may especially include a screening on one or more diseases selected from the group consisting of bone cancer, Osteomyelitis, and a transmittable disease. The bone part is only further processed, with the herein described (recycling) process, when the screening is negative on such bone disease, i.e. the bone part has not suffered in its history from cancer and/or Osteomyelitis, and/or a transmittable disease. In a specific embodiment, the screening includes a screening on one or more diseases based on one or more of a medical file and a medical interview of a human, wherein the one or more diseases which are screened are selected from the group consisting of bone cancer, Osteomyelitis, and a transmittable disease, and wherein the bone part is only further processed when the screening is negative on the one or more diseases (i.e. the bone part can be considered substantially free from bone cancer and/or Osteomyelitis deterioration). Of course, the screening may also include a screening on one or more other aspect, like bone fractures, osteoporosis, age of the donor, or infections with baro-resistant pathogens.

Screening is especially done before removal of the bone part from the donor body, although optionally screening may be executed after removal of the bone part from the donor body. Optionally, before and after removal the bone part may be screened. Especially however, at least one of these screenings, especially a screening before the bone part removal, include a screening on one or more diseases selected from the group consisting of bone cancer, Osteomyelitis, and a transmittable disease. The transmittable disease may e.g. be selected from the group consisting of HIV (human immunodeficiency virus), AIDS (acquired immunodeficiency syndrome), SARS (Severe Acute Respiratory Syndrome), MERS (Middle East Respiratory Syndrome), etc.. As will be clear to a person skilled in the art, only bone parts that pass the screening will further be processed. Hence, when a donor is not considered to be suitable as bone donor, the bone part will not be removed and processed as described herein. The phrase "screening a bone part (optionally external from a (donor) body)" and similar phrases may also be interpreted as screening a human or animal on suitability to be a bone (part) donor. Screening may e.g. be performed conform EU Guideline 2006/17/EC, Annex I SELECTION CRITERIA FOR DONORS OF TISSUES AND/OR CELLS (EXCEPT DONORS OF REPRODUCTIVE CELLS) AS REFERRED TO IN ARTICLE 3(a): 2.2. Allogeneic living donor, with 2.2.1: Allogeneic living donors must be selected on the basis of their health and medical history, provided on a questionnaire and through an interview performed by a qualified and trained healthcare professional with the donor, in compliance with point 2.2.2. This assessment must include relevant factors that may assist in identifying and screening out persons whose donation could present a health risk to others, such as the possibility of transmitting diseases or health risks to themselves. For a donation, the collection process must not interfere with or compromise the health or care of the donor. In case of cord blood or amniotic membrane donation this applies to both mother and baby; and with 2.2.2: Selection criteria for allogenic living donations must be established and documented by the tissue establishment (and the transplanting clinician in case of direct distribution to the recipient), based on the specific tissue or cells to be donated, together with the donor's physical status and medical and behavioral history and the result of clinical investigations and laboratory tests establishing the donor's state of health.

Due to the present process, the (microbial) screening on bacterial contamination in combination with the present process can lead to a much more certain (safe) product, which is not (microbially) contaminated at all or at least has a substantially decreased bioburden (after processing). Hence, the invention provides a bone part and/or bone reconstruction material having a low bio burden (for the recipient). Alternatively or additionally, as the present high pressure process is very efficient in eliminating the bacteria, even the microbial screening on bacterial contamination may be omitted at all. Bacterial contamination may e.g. occur at an operating theater or operating room (OR) during or after the surgeon has removed a bone part. With the present process, this superficial bacterial contamination that may occur at the OR (operating room) is not a problem anymore, as the high pressure process substantially eliminates the bacteria. Further, it appears that with the present high pressure process at the herein indicated conditions, the bone parts are not substantially deteriorated and remain their strength; lamellas may remain intact, thereby allowing succesfull ingrowth of new bone cells.

Further, with the present process bone parts can efficiently be processed into bone material that can be reused as bone reconstruction material. Hence, in a further aspect the invention also provides bone reconstruction material, for instance for use in a medical treatment wherein for instance an (undesired) defect in a bone of a human or animal is at least partly filled with the bone reconstruction material, wherein the bone reconstruction material is obtainable by the process as defined herein, especially wherein the bone reconstruction material has been obtained by the process as defined herein. As the bone reconstruction material may in an embodiment be especially particulate, the defect may be at least partly filled with the bone reconstruction material, optionally in combination with other material known to the person skilled in the art. In a specific embodiment, the invention provides a bone reconstruction material, for use in a medical treatment, wherein an undesired defect in a bone of a human or animal is at least partly filled with the bone reconstruction material, wherein the bone reconstruction material is obtainable by the process as defined herein, especially wherein the bone reconstruction material has been obtained by a process comprising (i) screening a bone part on the suitability for reuse in a human body, (ii) packaging the bone part, (iii) subjecting the bone part to a high pressure process, wherein the bone part enclosed by a package, wherein especially the packages is impermeable to water, is subjected to a pressure selected from the range of 450-800 MPa for a period of time selected from the range of 1-60 minutes, wherein the package especially comprises a closed polymeric package containing the bone part and an aqueous liquid, and (iv) optionally further packaging the thus obtained high pressure process processed bone part, wherein the bone part has one or more dimensions selected from the range of 1-65 cm, wherein the method may further comprise (v) optionally transporting the packaged bone part to a processing stage, and (vi) removing the package and optionally disintegrating at least part of the bone part for use as said bone reconstruction material. Hence, the medical treatment may especially include a partial or complete bone reconstruction. Especially, the bone reconstruction material may comprise the bone part or an at least partly disintegrated bone part. The bone part (especially after processing) may also be disintegrated to particulate material. Hence, the invention also provides (particulate) bone filler material, which is herein also indicated as (particulate) bone reconstruction material.

Hence, the invention also provides (particulate) bone filler material. A surgeon, desiring to use this bone reconstruction material may therefore at an operating room, during the process of operating a patient, (especially after opening the package) disintegrate at least part of the bone part into the bone reconstruction material. As the surgeon can open the packaged bone part at the operating room, a (new) contamination is prevented or the chance thereon is at least reduced in comparison to using unpackaged bone parts or using bone parts provided with state of the art processes. Further, as the patient is under operation, the surgeon can evaluate what and/or how much bone reconstruction material may be necessary (how it should fit). Hence, the present invention provides high flexibility to the surgeon, who can customize the bone part to the desired bone reconstruction material at the OR. As indicated above, the bone reconstruction material may in an embodiment be used as bone filler material (for filling a defect).

Different type of bone parts may be used. The term "bone part" may refer to an integral bone, like a skull, a humerus, an ilium, a radius, an ulna, a femur, a fibula and a tibia, etc., which are herein also indicated as bone element. However, also parts thereof may be used, such as a femoral head, etc. In a specific embodiment, the bone part comprises a femoral head. For instance, the bone part has one or more dimensions selected from the range of 1-65 cm. Such dimension may especially be the length, such as the (entire) length of a humerus, a radius, an ulna, a femur, a fibula or a tibia. One or more of the dimensions of the bone part may of course also be smaller, such as in the range of 1-10 cm. Especially, the smallest dimension, such as length, width, and thickness, are at least 0.5 cm, even more at least 1 cm, such as at least 1.5 cm. However, other dimensions may also be possible.

Hence, the term "bone part" may refer to an integral bone (bone element) or part thereof. This bone part, after removal of the body, is treated with the herein described high pressure process, to provide the reconstruction material. The reconstruction material may thus refer to the treated bone part, as well as treated disintegrated material, disintegrated before or after the high pressure process. The disintegrated material may especially be filler material. Disintegration may also be done before high pressure processing. In such instance the disintegrated bone material is subjected to the high pressure process, and reconstruction material, especially bone filler material, is obtained.

Hence, especially the bone part is used without a pretreatment that changes the bone part. The bone part, after removal from the body and (optionally) taking the nescesary samples for microbial screening, can be packaged directly in a polymeric bag for further HPP or the bone part can be superficially cleaned (c.q. rinsed) to remove any excess material before packaging in the polymeric bag for further HPP treatment. However, especially the bone part is not subjected to a demineralization and/or decellularization stage, especially neither of these.

Herein, the invention is especially explained with respect to (the processing of) bone parts, with optional disintegration after HPP. However, the invention is not limited to (HPP treatment of) bone parts only. However, the (HPP) processing conditions, etc. described below may also apply to bone material disintegrated (into particulate bone material) before HPP.

In a specific embodiment, the bone part is subjected to the pressure of at least 500 MPa, such as selected from the range of 550-750 MPa, for a period of time, especially selected from the range of 2-30 minutes, such as at least three minutes, like 3-15 minutes. Especially under these conditions there seem to be an optimum in time, pressure, bone properties and efficiency. These times relate to the times the bone part is under this pressure, and does (thus) not include the pressure increase and pressure decrease time. As indicated above, these conditions may also apply to the process wherein disintegrated material (or particulate bone material) is processed.

During the high pressure process, due to the increase in pressure the bone part may heat up. It advantageously appears that it is not necessary to compensate for this temperature increase with increasing pressure. Hence, especially during subjecting the bone part to the pressure the bone part only heats up adiabatically. However, one may desire to prevent too high temperatures. Hence, in a specific embodiment the bone part is kept at a temperature of at maximum 50 °C, even more especially below 40 °C, especially at a temperature of 37 °C or lower, though especially also not lower than about 5 °C.

Especially, before the high pressure is applied to the bone part, the bone part may be packaged. The package may optionally include more than one bone part. The term "bone part" may herein also refer to a plurality of bone parts. The term "bone element" may herein also refer to a plurality of bone elements. An advantage of packaging is that after HPP (or mild pasteurization), the bone part is not subjected again to a non-sterile environment. The package is thus closed (e.g. by a seal), and is substantially impermeable to water as during the application of the high pressure, a pressure chamber may contain a liquid to transfer the pressure to the bone part. It advantageously appears that the package itself may also include a liquid. This (package) liquid may comprise water, or another aqueous liquid, such as a physiological salt solution (or saline or buffer solution, especially a PBS (phosphate buffered saline)). In a further embodiment, the package may (thus) include only the bone part(s) and the aqueous liquid. Hence, especially the aqueous liquid is water, or another aqueous liquid, such as a physiological salt solution (or saline or buffer, or buffered saline solution), but the liquid does not include further additives. Hence, in an embodiment the package includes a liquid selected from water and a physiological salt solution. Therefore, the contents of the package may be the bone part and the liquid selected from a physiological salt solution, or a buffer solution, or water (wherein the term "bone part" may thus also refer to a plurality of bone parts). The (package) liquid may thus consist of a liquid especially selected from the group consisting of water, a physiological salt solution, and a buffered liquid. Especially, the (aqueous) liquid does e.g. not contain micro particles of titanium and silver). Especially, the liquid is an aqueous liquid. As buffer solution, e.g. a solution like a 0,1 M PBS pH 7,4 or any other biological buffering solution like for example a Tris, PIPES, HEPES, Tricine or a Trizma solution. The pH is especially in the range of 6.6-8, such as 6.8-8. The aqueous liquid especially may also comprise water, a buffer solution and a saline solution. PBS, for instance especially is a buffered saline solution comprising water, a buffer and saline solution. Hence especially the (package) liquid may comprise a liquid, especially (one or more liquids) selected from water, a saline solution and a buffer solution.

The package especially comprises a polymeric package, i.e. the envelope or package is of a polymeric material, such as PP or PE. Hence, in an embodiment the packaging comprises a closed polymeric package containing the bone part and an aqueous liquid. For instance, the closed polymeric package may be a plastic bag. Especially, the closed polymeric package is a flexible package. The volume of liquid relative to the bone part is especially in the range of 1-200%, especially 10-100% (relative to the bone part) (with 100% being 1:1 volumes of the liquid and the bone part, and with 10% being e.g. a bone part having a volume of 100 ml and the liquid in the package having a volume of 10 ml).

After the high pressure, the bacterial presence that may lead to an infection or other undesired effects may be substantially absent. Hence, in yet a further aspect the invention also provides a packaged bone part, especially a human bone part, comprising a (polymeric) package enclosing a bone part and an aqueous liquid having a CFU/ml of especially less than 10, such especially less than 1, such as 0.1, such as even more especially less than 0.01, though lower values, like a CFU/ml of less than 0.001 are also possible. In general, no activity was found at all after the treatment. As indicated above, the aqueous liquid comprises a physiological salt solution, or buffer solution or water. In most experiments the remaining activity was below the detection limit of 0.01 CFU/ml. Only an artifact (contamination at the stage of the microbial analysis by a not in our experiments used microbial strain) resulted in another result for the most sensitive analysis resulting in an other lower detection limit for that specific analysis.

A further advantage over prior art solutions is that the bone part obtained with the present process can be stored at room temperature, or can at least be provided to an operating room at room temperature, whereas prior art solutions store the bone parts at relative low temperatures, such as at a temperature lower than -30 °C, such as -80 °C, or even at liquid nitrogen temperature. Hence, the present invention provides an increased flexibility for the surgeon, as it may eliminate the thawing procedure which is normally being performed in theater (operating room), possibly even saving time in theater next to the increased flexibility. For instance, more bone material may be at the surgeons disposal during operation (for use in reconstruction), because of the increased shelf life at room temperature or moderate cooling conditions, such as in the range of 0-20 °C, such as 2-10 °C. Hence, whereas prior art methods include deep freezing for maintaining shelf life, with the present invention the shelf life is even high at very moderate cooling temperatures (even not frozen), allowing maintaining more material at the shelf (in the hospital). The term "shelf life" is especially defined as in the art, and is especially defined as the length of time the bone part may be stored without becoming unsuitable for use (i.e. here optional partial disintegration and use as reconstruction material (in a human or animal body)). Hence, the process may further include storing the bone part at a temperature in the range of 0-20 °C, especially in the range of 2-10 °C. Therefore, the process may include maintaining the shelf life at a cooling temperature wherein the bone part is not frozen, such as in the range of 0-20 °C, especially in the range of 2-10 °C. Storage in the range of 0-20 °C, especially in the range of 2-10 °C, is especially done after the application of the high pressure. Further, the packaged bone part may be stored at these temperatures until about the removal of the package. Hence, also during transporting the packaged bone part to the processing stage, the bone part may be stored at these temperatures. Hence, the relative high temperature at which the (packaged) bone part may be stored is advantageous in view of transport and also in view of storage. Further, it facilitates the use in medical applications, as there is (substantially) no thawing time. Storage management may thus be much easier.

In case the bone part is not packaged before applying the pressure, the bone part may be packaged after applying the pressure. In such instance, a liquid is not necessarily available in the package, though may optionally still be included. Further, optionally a packaged bone part that has been processed with the high pressure may be included in a second package, e.g. for safety reasons, like protective reasons. Hence, the phrase "optionally (further) packaging" may refer to a first package after HPP of a bone part that was not packaged, or to an optional (second) packages of bone part that has already been packaged before HPP.

Especially, a first package enclosing the bone part is especially impermeable to water. During the HPP, the package is especially entirely submerged in a liquid in a pressure chamber, as the pressure chamber may contain a liquid to transfer the pressure to the (packaged) bone part.

After the high pressure process there may be a further screening on possible damage of the bone part and/or the optional package. In general, the site where the high pressure process is executed is another site than the operating room. Hence, the packaged bone part may be transported (with means known in the art) to the operating room. Of course, there may be one or more intermediate storages, such as at the site of the high pressure process, at the site of the operating room, and optionally in between (distribution center, etc.).

When disintegrating the bone part (see also above), this may be done to obtain bone powder and/or larger bone particles. This material may be used as reconstruction material (see also above). Especially, at least part of the bone part is (subsequent to the high pressure process and optional packaging) processed into particulate bone material having dimensions selected from the range of 0.1-5 mm, though other dimensions may also be possible, for use as bone filler material. For instance, at least 75 wt.% of the particulate bone material may have these dimensions. In an embodiment, the bone reconstruction material is selected from the group consisting of autograft, allograft or xenograft material. The defect that has to be at least partly filled with the bone filler material may in an embodiment be a man-made defect. For instance, in embodiments the defect is the result of a removal of an implant or of an implant fastening element. The bone part may also be disintegrated into chips (after HPP or optionally before HPP). However, the defect may also be a fracture.

In another aspect, full disintegration may not take place, but the bone part, optional after physical adaptation of the bone part to the recipient (place) (in the body) may be arranged in the body. Hence, the invention also provides bone reconstruction material that may be used as implant or graft. Here, disintegration may include removal of e.g. pieces to provide the desired graft dimensions for implantation in the recipient.

The process of the invention does not include the removal of the bone part from a (living) donor. The process of the invention uses a bone part after removal from the (living) donor.

As indicated above, the invention provides especially a process, especially for the efficient reuse of human bone material, the process comprising (i) screening a bone part on the suitability for reuse in a human body, (ii) packaging the bone part, (iii) subjecting the bone part to a high pressure process, wherein the bone part enclosed by a package, wherein the package is impermeable to water, is subjected to a pressure selected from the range of 450-800 MPa for a period of time selected from the range of 1-60 minutes, and (iv) optionally further packaging the thus obtained high pressure process processed bone part, wherein the bone part has one or more dimensions selected from the range of 1-65 cm.

The term "substantially" herein, such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of". The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

The invention further applies to a device comprising one or more of the characterizing features described in the description and/or shown in the attached drawings. The invention further pertains to a method or process comprising one or more of the characterising features described in the description and/or shown in the attached drawings.

The various aspects discussed in this patent can be combined in order to provide additional advantages. Furthermore, some of the features can form the basis for one or more divisional applications.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Fig.1a schematically depicts an embodiment of process of removing the bone part to arrangement of the bone reconstruction material; Fig. 1b schematically depicts some aspects of the invention.
Figs 2a-2b show experimental setups and additional information. The flow chart of fig 2a provided amongst others the results of table 1-2; the flow chart of fig. 2b provided amongst others the results of tables 3-4; and

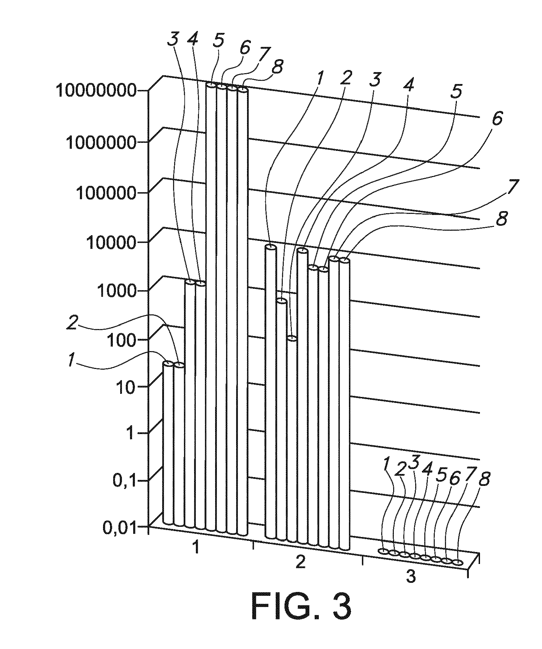

Fig. 3 shows some results. These data are from an test performed in duplicate test. On the x-axis at 1: real starting concentration (CFU/ml); at 2: concentration after transport without HPP (CFU/ml); and at 3: concentration after transport and HPP (CFU/ml). On the y-axis 100 of CFU/ml are indicated, i.e. the axis is from 1-1.10⁹ CFU/ml.

### DETAILED DESCRIPTION OF THE EMBODIMENTS

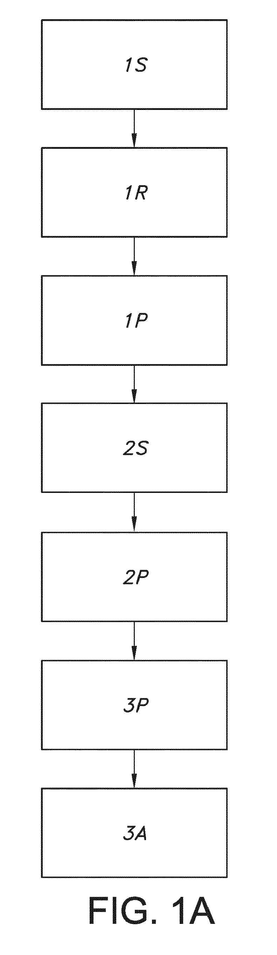

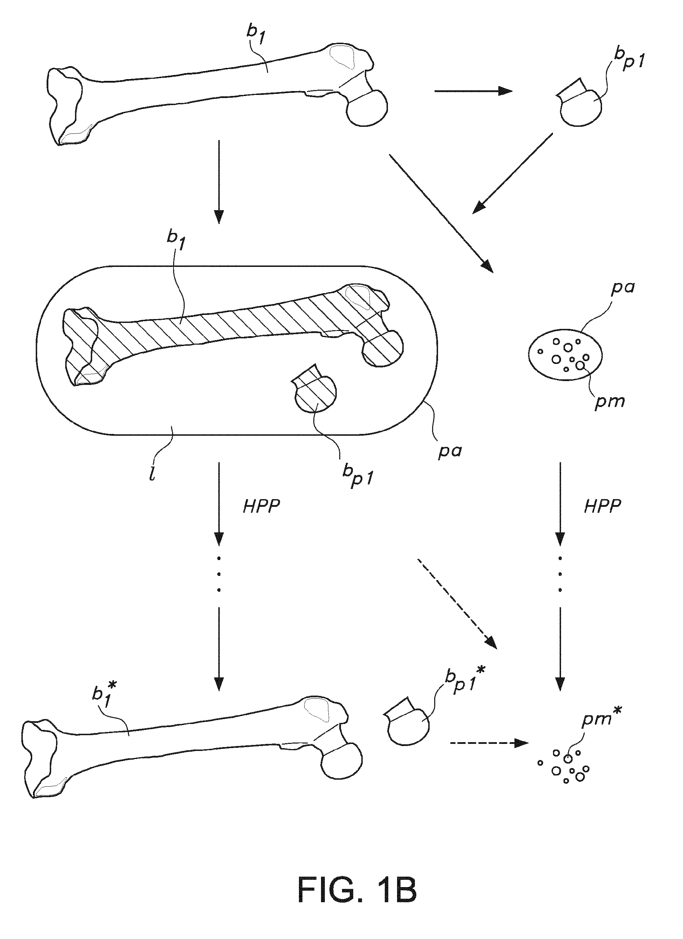

Fig.1a schematically depicts an embodiment process of removing the bone part to arrangement of the bone reconstruction material, starting at the removal from a body (stage 1R), a screening (1S), optional packaging (not shown), high pressure processing (stage 1P), optional screening (2s, e.g. on damage material and/or damaged packaging, or other problems), optional packaging (2P), transport or further processing, here only processing is indicated with stage 3P, which may optional include the disintegration of the bone part into reconstruction material or optional fine tuning to use as graft, and finally application (3A), such as applying as filler material or graft. Optional storage between two stages, which may be performed at different moments within the process (such as before the high pressure process 1P and/or (directly) after the high pressure process), is not indicated in this scheme. Optionally, the order between screening 1S and removal from the body may be reversed, i.e. a first screening on suitable bone donorship is done before the bone part(s) is (are) removed; this situation is in fact schematically depicted in fig. 1b. Intermediate storage may be done at temperatures below 0 °C, whereas optional in the hospital storage may be above 0 °C (see also above). This relative high temperature storage, enabled by the present process is of advantage for quick access to the surgeon.

Fig. 1b schematically depicts bone parts b1 (integral bone) and bp1 (a part of an integral bone), the former being an integral bone, and the latter thus being a part. This bone part b1,bp1, after removal of the body, is treated with the herein described high pressure process, to provide the reconstruction material. However, especially the bone part b1,bp1 may be packaged before treating with the high pressure process. The package is indicated with reference pa. Optionally, this package may be filled with a liquid L (such as the saline or buffer solution, or water). By way of example, the left package contains two bone parts. However, also only one or more bone parts may be available in the package pa.

After processing, indicated with the arrow HPP, the bone part b1,bp1 may be used as such, such as a graft, or may be disintegrated, indicated with the arrow to the right, to provide particulate material (particulate reconstruction material). Both are considered reconstruction material. The asterisk * indicates that the bone part b1,bp1 have been treated. Likewise this applies to the particulate material pm, which is indicated after treatment with pm*. When the bone part b1* or bp1* is used as such, still some disintegration may be performed to fit in the recipient.

The reconstruction material may refer to the treated bone part b1*,bp1*, as well as disintegrated material pm*, disintegrated after the high pressure process. The disintegrated material may especially be filler material. Disintegration may also be done before high pressure processing. In such instance the disintegrated bone material pm is subjected to the high pressure process (especially in a package pa), and particulate reconstruction material, especially bone filler material pm* is obtained. Herein, the invention is especially explained with respect to bone parts. However, the invention is not limited to bone parts only.

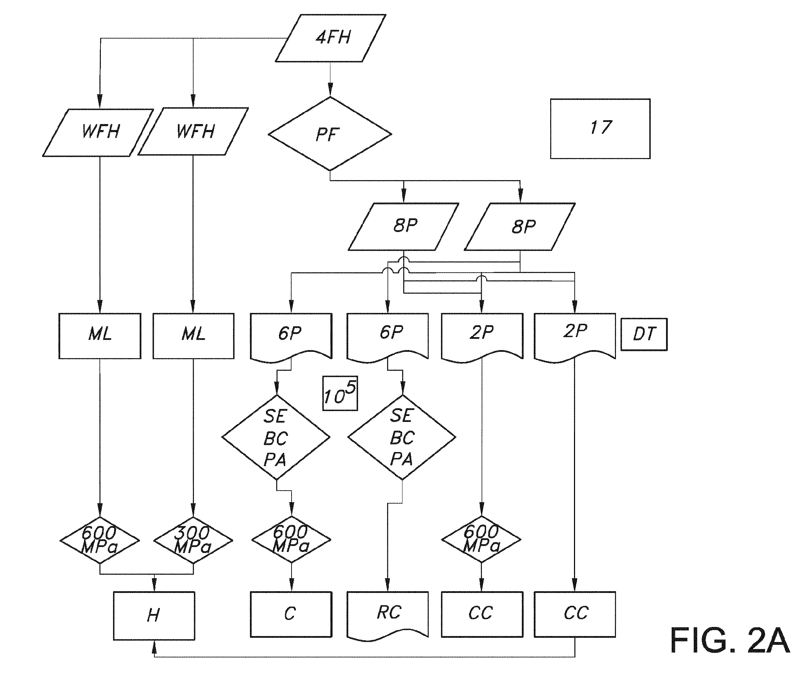

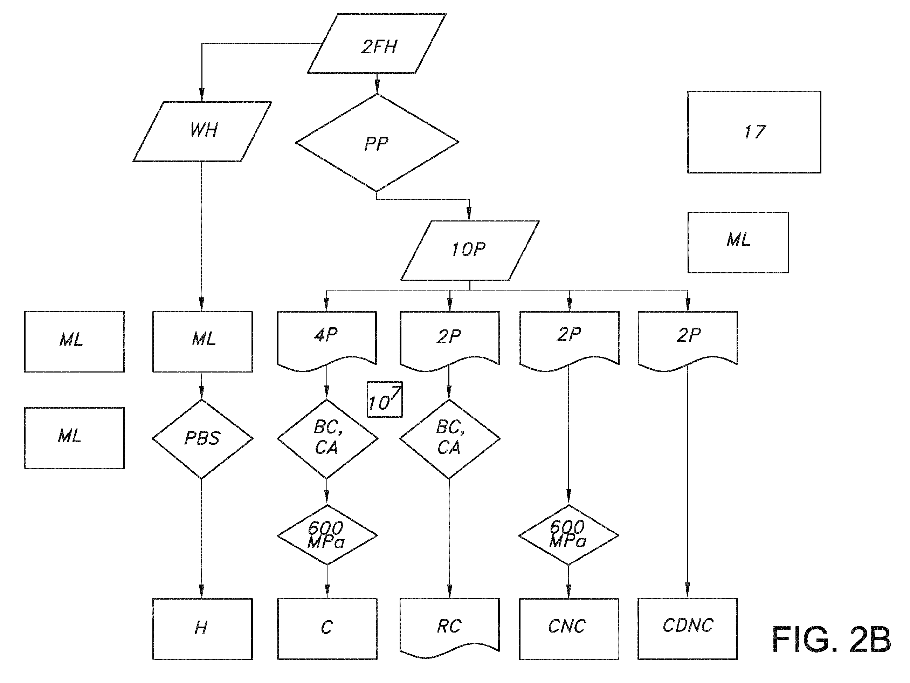

Fig. 2a shows a flowchart in relation to the experiments that were performed. The elements in the chart in figs. 2a and 2b are indicated in the table below:

| **Element in** **fig. 2a** **and** **2b** | **Description** |
|---|---|
| WFH | Whole femoral head |
| 4FH | 4 femoral heads |
| PF | Partitioning of 2 femoral heads in 16 parts (2a) |
| PP | Partitioning of 1 femoral heads in 10 parts (2b) |
| 17 | Using an oscillating saw under laminar flow in sterile conditions |
| 8P | 8 parts |
| ML | Microbiology lab |
| 6P | 6 parts |
| 2P | 2 parts |
| DT | in Duplicate Test |
| SE | *S. epidermidis* |
| BC | *B. cereus* |
| PA | *P. aeruginosa* |
| H | Histology |
| C | culture |
| RC | Reference culture |
| CC | Culture - control |
| 2FH | 2 femoral heads |
| WH | Whole (femoral) head |
| 10P | 10 parts |
| CNC | Culture negative control |
| 4P | 4 parts |
| CDNC | Culture double negative control |
| PBS | Phosphate buffered saline |
| 300 MPa | HPP at 300 MPa for 30 minutes. Other pressures (like 600 MPa) were applied for the same time. |

### EXPERIMENTAL

Seven fresh frozen femoral heads from a bone bank were selected. Donors and femoral heads were screened and cultured by standard; all negative. Four femoral heads were divided into 20 equal parts in surgical theater under down flow and sterile circumstances, using an oscillation saw. They were again packaged using a sterile container and then transported to the department for clinical microbiology, in a cooled container. There 16 parts were contaminated in duplicate in a sterile and later sealed plastic bag containing 100 ml of Phosphate-Buffered Saline (PBS) with 10⁵ CFU/ml of *Staphylococcus epidermidis, Bacillus cereus, Pseudomonas aeruginosa* and *Candida albicans,* respectively (Flowchart 1; fig. 2a). Four parts were not contaminated but directly packaged in 100 ml of the same Phosphate-Buffered Saline (PBS); pH 7.5, again in a sealed sterile plastic bag. Three additional intact femoral heads were left unharmed but underwent the same protocol with the exception of contamination.
1. Contaminated with *S. epidermidis,* to undergo HPP (in duplicate)
2. Contaminated with *S. epidermidis,* but no HPP (in duplicate)
3. Contaminated with *B. cereus*, to undergo HPP (in duplicate)
4. Contaminated with *B. cereus,* but no HPP (in duplicate)
5. Contaminated with *P.aeruginosa,* to undergo HPP (in duplicate)
6. Contaminated with *P.aeruginosa,* but no HPP (in duplicate)
7. Contaminated with *C.albicans,* underwent HPP (in duplicate)
8. Contaminated with *C.albicans,* but no HPP (in duplicate)
9. Negative "sterile" control; only bone fragment with PBS, to undergo HPP (in duplicate)
10. Negative control; No contamination and no HPP, only bone fragment with PBS (in duplicate)
11. Femoral head for microscopy, HPP at 300 MPa.
12. Femoral head for microscopy, HPP at 600 MPa.
13. Femoral head for microscopy, but no HPP

### S. Epidermidis, P.aeruginosa, B. cereus (see fi. 2a)

To obtain a starting concentration of the bacteria of 10⁵ CFU/ml, 1 ml of a concentration of 10⁷ CFU/ml was diluted in 99 ml PBS. Each contaminated sample was contaminated with 10⁵ CFU/ml. The femoral head parts were sealed in 100 ml PBS. 0.5 McFarland contains 1,5.10⁸ CFU/ml. Diluting these solutions 15 times, results in a start concentration of 10⁷ CFU/ml.

### C.albicans, B. cereus (see fig. 2b)

Each sample was contaminated with 10⁷ CFU/ml. Since the femoral head parts were sealed in 100 ml PBS, starting concentration of the bacteria was now increased to 10⁹ CFU/ml. From the starting concentrations, 1 ml was diluted in 99 ml to achieve a concentration of 10⁷ CFU/ml. The starting concentration was made using a Mcfarland 4 dilution.

The bags were vacuum-sealed and sent off under sterile and cool conditions to undergo HPP at a secondary location. After returning the following day, 1 ml of suspension was diluted in PBS (10⁶ - 10⁵ -10⁴ - 10³ - 10²) and 50 µl was cultured on TSS plate. From the undiluted samples 200 µl was plated out. All plates were incubated for 48 hours at 37°C. When the cultures remained negative, the femoral head and 100 ml PBS was mixed with 100 ml liquid BHI broth and incubated for 48 hours to detect low concentrations.

Of each bacterium *(S. Epidermidis, P.aeruginosa*), a 1 ml suspension was made using 0,5 McF. This suspension was diluted in 14 ml PBS. With this new suspension the femoral heads was contaminated by transferring 1 ml to the plastic bag containing the femoral head filled with 99 ml PBS. From this suspension, dilution series (1:10) were made of 10⁴ - 10³ - 10² to determine the precise inoculum. From the last tree dilution 50 µl was plated out on TSS plates and CLED medium. The number of CFU will be 500- 50-5 when the starting concentration is correct. The plates were cultured for 24 and 48 hours at 37°C.

The bags were all vacuum-sealed and preserved under cooled condition at 4-8°C and send off to undergo HPP. After HPP at 600 Mpa, the bags were transported to the measuring facility, again under sterile and cooled circumstances at 4-8°C.

All contaminated bags were opened and cultured in a sterile environment at the department of clinical microbiology. Cultures were performed by sampling 1 ml of suspension out of the bag and preparing dilution series in PBS (10⁴ - 10³ - 10² ). Subsequently, 50 µl of the dilutions was plated on blood agar plates, and 200 µl from the undiluted samples.

Dilution series (1:10) were made from the suspension containing *C.albicans, B. cereus,* varying from10⁶ - 10⁵ -10⁴ - 10³ - 10² to determine the precise inoculum. From the last tree dilutions, 50 µl was plated onto TSS plates (*B. cereus*) and SAB (*C.albicans*) medium. The plates were cultured for 24 and 48 hours at 37°C All the plates were incubated for 48 hours at 37°C. When the cultures remained negative after 48 hours, the femoral head and the 100 ml PBS were cultured in 100 ml liquid Brain Hart Infusion (BHI) broth in order to detect low concentrations by enrichment. After 48 hours, the BHI was sub cultured to examine growth of bacteria again.

### Results

**Table 1: Control starting concentration**

| **Bacteria** | **Dilution** | **Incubation 24 hours** | **Incubation 48 hours** | **Real starting concentration** |
|---|---|---|---|---|
| *Staphylococcus epidemidis* | 10⁴ | 100 CFU | 100 CFU | 0.2.10⁴ |
| | 10³ | 45 CFU | 45 CFU | 0.9.10³ |
| | 10² | 4 CFU | 4 CFU | 10² |
| *Bacillus cereus* | 10⁴ | 50 CFU | 50 CFU | 10³ |
| | 10³ | - | - | - |
| | 10² | - | - | - |
| *Pseudomonas aeruginosa* | 10⁴ | ±500 CFU | ±500 CFU | ±10⁴ |
| | 10³ | 55 CFU | 55 CFU | 1.1.10³ |
| | 10² | 5 CFU | 5 CFU | 10² |

**Table 2: Results after HPP at 600 MPa**

| | **Sample** | **femoral head** | **Growth on plate** | | | **CFU/ ml** | **BHI** | **Growth in BHI** |
|---|---|---|---|---|---|---|---|---|
| | | | 10⁴ | 10³ | 10² | | | |
| 1 | Stapep HPP | 1 | - | - | - | - | - | - |
| 2 | Bacice HPP | | - | - | - | - | - | - |
| 3 | Pseuae HPP | | - | - | - | - | - | - |
| 4 | Negative control HPP | | - | - | - | - | - | - |
| 5 | Negative control without HPP | | - | - | - | - | - | - |
| 6 | Stapep without HPP | | ±500 | 50 | 7 | 10⁵ | - | - |
| 7 | Bacice without HPP | | - | - | - | - | - | - |
| 8 | Pseuae without HPP | | ±700 | 70 | 17 | 1.4.10⁴ | | |
| 9 | Stapep HPP | 2 | - | - | - | - | - | - |
| 10 | Bacice HPP | | - | - | - | - | - | - |
| 11 | Pseuae HPP | | - | - | - | - | - | - |
| 12 | Negative control HPP | | - | - | - | - | - | - |
| 13 | Negative control without HPP | | - | - | - | - | - | - |
| 14 | Stapep without HPP | | ±400 | 38 | 14 | 0.8.10⁵ | | |
| 15 | Bacice without HPP | | - | - | - | - | Turbid | Pseuae |
| 16 | Pseuae without HPP | | ±500 | 70 | 10 | 10⁵ | | |

### Results

**Table 3: Control starting concentration**

| Bacteria | Dilution | Incubation 24 hours | Incubation 48 hours | Real starting concentration |
|---|---|---|---|---|
| *Candida albicans* | 10⁴ | 500 CFU | 500 CFU | 10⁹ |
| | 10³ | 45 CFU | 45 CFU | |
| | 10² | 4 CFU | 4 CFU | |
| *Bacillus cereus* | 10⁴ | +/- 500 CFU | +/- 500 CFU | 10⁹ |
| | 10³ | 39 CFU | 39 CFU | |
| | 10² | 15 CFU | 15 CFU | |

**Table 4: Results after HPP**

| | Sample | Growth on plate | | | CFU/ml | Growth in BHI |
|---|---|---|---|---|---|---|
| | | 10⁴ | 10³ | 10² | | |
| 1 | C.albicans HPP | - | - | - | - | - |
| 2 | C.albicans HPP | - | - | - | - | - |
| 3 | C.albicans without HPP | 200 | 15 | 4 | 0.5.10⁷ | + |
| 4 | B.cereus HPP | - | - | - | - | - |
| 5 | B.cereus HPP | - | - | - | - | - |
| 6 | B.cereus without HPP | +/- 500 | 40 | 3 | 0.8.10⁷ | + |
| 6 | B.cereus without HPP | +/- 500 | 40 | 3 | 0.8.10⁷ | + |
| 7 | Negative control HPP | - | - | - | - | - |
| 8 | Negative control HPP | - | - | - | - | + |
| 9 | Negative control without HPP | - | - | - | - | - |
| 10 | Negative control without HPP | - | - | - | - | - |

The positive value in sample 8 appeared to be an artefact.

### Histology

The three intact femoral heads were analyzed with microscopy using Hematoxylin and eosin (HE) staining (figs 2a and 2b).

All three were packaged in a sterile environment and stored in a plastic bag, sealed in 100 ml PBS. One was left untreated, the other two underwent either HPP with 300, 600 MPa and all were stored for 5 days. After 5 days the femoral heads were fixated with formalin and were decalcified. After 5-7 days the femoral heads were cut in half and the central part of the femoral head was then cut to be analyzed using HE staining.

Nuclear staining of the osteocytes clearly decreased with every step; leaving potentially viable osteocytes in there lacuna in the untreated femoral head, some after 300 MPa and almost none 600 MPa . Microscopic bone structure assessed within the osteon as the number of intact lamellae appeared unchanged for all three femoral heads.

The hypothesis was that HPP resulted in a log 4 to 5 reduction of the amount of bacteria administered. Looking at these results the hypothesis is correct for *Staphylococcus epidermidis* and *Pseudomonas aeruginosa.* Both bacteria were not present after HPP, which resulted in a log 5 reduction. HPP resulted in a log 3 reduction of *B. cereus.*

Sample 15 was contaminated with *Pseudomonas aeruginosa,* occurring when the growth medium BHI was cultured.

Our results show at least a minimal log 4 to 5 reduction of the amount of Bacillus and yeast administered. We found a log 7 reduction for all microorganisms used in the follow up experiment for the contamination of the allograft. The control of the starting concentration confirmed that the administered concentration was correct. The control bags that didn't underwent HPP treatment, showed growth as expected. The concentration after transport was somewhat lower than the starting concentration.

Herein, the following abbreviations are applied:

| |
|---|
| Stapep: *Staphylococcus epidermidis* |
| Bacice: *Bacillus cereus* |
| Pseuae: *Pseudomonas aeruginosa* |
| CFU: Colony forming units |
| McF: MacFarland |
| HPP: High Pressure Processing |
| BHI: Brain Heart Infusion |
| PBS: Phosphate-Buffered Saline |
| SAB: special medium for Yeast |

Hence, amongst others four femoral heads were divided into 20 parts, of which 16 were contaminated (duplicate) in a sterile plastic bag containing 100 ml of PBS with 10⁵ CFU/ml of *Staphylococcus epidermidis, Bacillus cereus, Pseudomonas aeruginosa* and *Candida albicans,* respectively. Of each duplicate, one sample was untreated and stored similarly as the treated sample. The remaining 4 parts were included as sterile control and non-infected control for histological examination. The parts in plastic bags were vacuum sealed and underwent HPP at the high pressure value of 600 MPa. After HPP, serial dilutions from the plastic bags were made and cultured on selective media and into enrichmnent media (Brain Heart Infusion Broth) to recover low amounts of microorganism and spores. Cultures were incubated for 48 hours at 37 C. The colony forming units of the inocolum were corrected for the cultured colonies from the untreated contaminated bags. Three additional complete femoral heads were threated with 0, 300 and 600 MPa HPP respectively and were assessed with histological examination. None of the sterile control bone fragments contained microorganisms. The measured colony counts correlated excellent with the expected colony count. All HPP treated bone fragments did not grow on culture plates or enrichment media, expect for one *B. cereus* growth control which revealed *P. aeruginosa.* The experiment was repeated for *B. cereus* combined with higher start inocolum of 10⁷ CFU and resulted in complete sterilization and appropriate controls. Histological examination showed Nuclear staining of the osteocytes clearly decreased with every step; leaving potentially viable osteocytes in there lacuna in the untreated femoral head, some after 300 MPa and almost none 600 MPa. Microscopic bone structure assessed within the osteon as the number of intact lamellae appeared unchanged for all three femoral heads.

## Claims

1. A process, especially for the efficient reuse of human bone material, the process comprising (i) screening a bone part on the suitability for reuse in a human body, (ii) packaging the bone part, (iii) subjecting the bone part to a high pressure process, wherein the bone part enclosed by a package is subjected to a pressure selected from the range of 300-1000 MPa for a period of time selected from the range of 1-60 minutes, wherein the package comprises a closed polymeric package containing the bone part and an aqueous liquid, and (iv) optionally further packaging the thus obtained high pressure process processed bone part, wherein the bone part has one or more dimensions selected from the range of 1-65 cm.

2. The process according to claim 1, wherein the bone part is a bone part of a living donor.

3. The process according to any one of the preceding claims, wherein the pressure is selected from the range of 450-800 MPa, the process further comprising (v) transporting the packaged bone part to a processing stage, and (vi) removing the package and disintegrating at least part of the bone part for use as bone reconstruction material.

4. The process according to any one of the preceding claims, wherein the screening includes a screening on one or more diseases based on one or more of a medical file and a medical interview of a human, wherein the one or more diseases which are screened are selected from the group consisting of bone cancer, Osteomyelitis, and a transmittable disease, and wherein the bone part is only further processed when the screening is negative on the one or more diseases.

5. The process according to any one of the preceding claims, wherein the screening includes a screening on one or more diseases based on a medical interview of a human, wherein the human is the donor to be of the bone part.

6. The process according to any one of the preceding claims, wherein the bone part comprises a femoral head.

7. The process according to any one of the preceding claims, wherein the bone part is subjected to a pressure selected of at least 500 MPa for a period of time selected from the range of 2-30 minutes, and wherein during subjecting the bone part to the pressure the bone part only heats up adiabatically.

8. The process according to claim 7, wherein the bone part is kept at a temperature of at maximum 37 °C.

9. The process according to any one of the preceding claims, wherein at least part of the bone part is processed into particulate bone material.

10. The process according to any one of the preceding claims, wherein the packaging comprises a closed polymeric package containing the bone part and an aqueous liquid, wherein said aqueous liquid consists of a liquid selected from the group consisting of a physiological salt solution, a buffer solution, and water.

11. Bone reconstruction material, for use in a medical treatment, wherein an undesired defect in a bone of a human or animal is at least partly filled with the bone reconstruction material, wherein the bone reconstruction material has been obtained by a process comprising (i) screening a bone part on the suitability for reuse in a human body, (ii) packaging the bone part, (iii) subjecting the bone part to a high pressure process, wherein the bone part enclosed by a package is subjected to a pressure selected from the range of 300-1000 MPa for a period of time selected from the range of 1-60 minutes, wherein the package comprises a closed polymeric package containing the bone part and an aqueous liquid, and (iv) optionally further packaging the thus obtained high pressure process processed bone part, wherein the bone part has one or more dimensions selected from the range of 1-65 cm.

12. The bone reconstruction material according to claim 11, wherein the bone reconstruction material is selected from the group consisting of autograft, allograft or xenograft material, wherein the defect is a man-made defect, and wherein the defect is the result of a removal of an implant or of an implant fastening element.

13. The bone reconstruction material according to any one of claims 11-12, wherein the process further comprises (v) optionally transporting the packaged bone part to a processing stage, and (vi) removing the package and disintegrating at least part of the bone part for use as said bone reconstruction material, and wherein the pressure is selected from the range of 450-800 MPa.

14. A packaged bone part, comprising a polymeric package enclosing a bone part and an aqueous liquid having a CFU/ml of less than 10, especially having a CFU/ml of less than 1.

15. The packaged bone part according to claim 14, wherein the aqueous liquid comprises a physiological salt solution, or a buffer solution, or water, and wherein the packaged bone part is obtainable by the process according to any one of claims 1-10.
